# EUROPEAN PATENT SPECIFICATION

(11) **EP 2 294 980 B1**
(45) Date of publication and mention of the grant of the patent: **28.03.2012**
(21) Application number: 10175817.5
(22) Date of filing: 08.09.2010
(51) Int. Cl.: A61B 5/103, A61F 2/46

(54) **Hip surgery assembly**
Hüftoperationsanordnung
Ensemble de chirurgie de la hanche

(30) Priority: 09.09.2009 NL 1037265
(43) Date of publication of application: 16.03.2011
(73) Proprietor: In Novation B.V., 1055 MC Amsterdam (NL)
(72) Inventor: De Wekker, Erwin, 1055 MC, Amsterdam (NL)
(74) Representative: De Vries & Metman

(56) References cited:
- EP-A1- 2 067 443
- EP-A2- 1 920 713
- WO-A2-03/096870
- WO-A2-2008/118524
- FR-A- 910 078
- US-A- 3 262 452

## Description

### TECHNICAL FIELD

The present disclosure pertains to the field of surgery assistance apparatus, in particular to apparatus for assisting hip-replacement surgery.

### BACKGROUND

Due to aging, disease or high load, patients may suffer from wear of joints (arthrosis). A common affliction is wear of the hip joint, in particular wear of the cartilage of the joint between the femoral head (head of the upper leg bone; convex part of the hip joint) and the interior of the acetabulum (bilateral cavity of the pelvis; concave part of the hip joint). Interaction and friction between the bare bone causes pain during movement. The pain and associated restriction in functionality further causes the patient to adapt its pattern of motions, in particular in walking. This again may lead to reactive pain in muscles and ligaments of the patient. Dependent on the amount of wear of the hip joint a total hip replacement operation may be performed. Goals of such operation are to enable pain-free movement and optimal regaining of active life prior to appearance of the symptoms.

In total hip replacement surgery both the femoral head and the acetabulum are replaced by prostheses. After resection of the worn femoral head a rod-like prosthesis is placed in the femoral shaft, usually made of titanium or cobalt-chrome which is provided with a ceramic or cobalt-chrome head, to replace the femoral head. Further, the interior side of the acetabulum is reamed and fitted with a acetabulum cup prosthesis, or cup for short, generally comprising polyethylene, titanium and/or ceramic material. The acetabulum cup replaces the original acetabulum. Both prostheses together form the new prosthetic joint.

Correct placement of both prostheses is crucial. Malpositioning can lead to a number of post-operative complications such as impingement (contact of the components in non-desired locations as a consequence of motion) and limitation of the operative range of motion of the hip joint. Further, non-physiologic (directions of) forces and wear may accelerate wear and possibly detachment of (constituent parts of) the prosthetic joint. Such complications may therefore lead to hip-luxation.

Luxation is usually caused by a malpositioned acetabulum cup. In most cases the only solution is revision (restoration) surgery, in which the primary cup is replaced with a new cup. In order to avoid possible second malpositioning extra attention is required for proper positioning and placement of the new cup.

Aside from common risks associated with surgery - in particular of elder and/or delicate patients - an additional risk in cup revision surgery is that less bone is present for a good grip of the cup because usually significant amounts of bone material have been reamed away for fitting the primary cup. In almost all cases, this means longer duration of surgery, therefore additional risk of infections, and additional uncertainties for both the patient and surgeon. It is clear that positioning and placement of a cup should be done very carefully in both primary and revision surgery. For correctly positioning a cup it is well known that the angles of anteversion and inclination are determining factors. The angle of inclination is defined with respect to a frontal plane. The anteversion angle is defined with respect to a sagittal plane.

Despite a lack of consensus amongst orthopaedic surgeons on the correct value of these angles, a safe value for the angle of anteversion is generally considered to be between about 10 and 25 degrees with a preferred value of about 20 degrees. For the angle of inclination a range of about 35 to about 50 degrees is considered safe, with a preferred value of about 45 degrees. The preferred values allow a clinically acceptable margin of error.

Many orthopaedic surgeons are unaware that the colloquial anteversion angle can be divided in a true anteversion angle and a planar anteversion angle. The true anteversion angle is defined with respect to a pure sagittal plane and is independent of the position of the cup. The planar anteversion angle is defined in a plane perpendicular to the cup, the reference system thus is dependent on the position and orientation of the cup. Similar holds for true and planar inclination angles. See for more information e.g. L. Fabeck et al, "A method to measure acetabular cup anteversion after total hip replacement", Acta Ortopaedica Belgica 65(4), 485-91 (1999). In other words, a variation in the angle of inclination affects the measured angle of planar anteversion.

Known instruments for measuring on and/or placing an acetabular cup do not distinguish or even allow for the distinguishing between true or planar anteversion angles.

Thus reference systems for determining correct orientation and position of a cup may be inaccurately defined and/or differently defined between different medical practitioners. Predictability and reproducibility are therefore adversely affected.

As reproducible surgery results are only possible with respect to well defined reference systems, the position of the pelvis before and during surgery needs to be known. The position may be determined with respect to palpable portions of the pelvis (e.g. spina iliaca anterior superior dextra and -sinistra and os pubis). During surgery the position of the pelvis may be obscured by surgical drapes. Changes of the position with respect to reference points defined beforehand may therefore become inaccurate during surgery; this may go unnoticed to the surgeon. A reliable reference system and reliable determination of the actebular position and orientation with respect to such reference system are therefore desired.

An existing system described in US 6.623.488 allows checking for movement of the patient in one direction only, and requires repositioning of the patient during operation to the initial position which is cumbersome, failure-prone and impossible for some patients and/or surgical procedures.

Systems according to US 2009/0105714 or US 2004/0210233 require sophisticated navigation instruments and computers which render the system complex and expensive. During surgery personnel may further be required to be conscious about blocking radiation and/or communication between (different parts of) navigation instruments, such that operation of the system interferes with (concentration on) the surgical procedure.

The hip endoprothesis implantation accessory described in US 2005/0107799 allows measuring an angle for implantation with respect to a reference object. However, no solution is provided for accurately and correctly orienting the accessory and/or the reference object with respect to a desired and/or predetermined orientation for the acetabular cup prosthesis. Furthermore, the described method requires initially reconstructing the femoral head, which most surgeons prefer to do after positioning the acetabulum cup since the femoral head may obscure parts of the surgical space and hinder subsequent procedures.

US 2008/0132903, see preamble of claim 1, discusses a goniometer for measuring artificial acetabular cup angles and a method for measuring thereof using the goniometer. The document distinguishes between operative anteversion and inclination angles on the one hand and radiographic anteversion and inclination angles on the other hand. The goniometer comprises two measurement units (defined in the document with 130 and 140 respectively) allowing establishing two angles in one operation. The document discloses that the first angle measurement unit (130) is configured to measure an inclination angle, the second angle measurement unit (140) is configured to measure an anteversion angle. However, upon closer inspection the second angle measurement unit is prone to introducing errors and proves only able to measure the planar angle of anteversion. Furthermore the goniometer is complex, unwieldy and clutters up the surgical space and hindering accuracy.

As a consequence there is a desire for apparatus for improving hip surgery addressing one or more of the aforementioned problems.

### SUMMARY

In order to provide such apparatus an assembly according to claim 1 is provided. Preferably the goniometer is configured such that the first axis of rotation is arrangable at least parallel to the axis of extension, preferably also perpendicular to the axis of extension. For increased freedom of operation, the first axis of rotation is arrangable substantially continuously between being perpendicular and parallel to the axis of extension. The goniometer allows to measure an orientation of the axis of extension of the surgical tool in a reliable manner with respect to a desired plane, as will be set out in more detail below. The first axis of rotation may be arranged substantially in any direction to be normal to the desired plane of measuring, such that a substantially pure measurement may be performed and the risk of determining projections, rather than actual angles is reduced or even prevented. The axes of rotation are separated such that relatively well-defined rotations may be performed, opposite to for example a ball-joint with which it is extremely hard to perform a rotation in a single plane, and not accidentally also perform a rotation in another direction, i.e. about another axis of rotation or an effectively tilted axis of rotation. Such inexact determination is a source of errors, e.g. incorrect identification of planar and true anteversion angles as indicated above. In addition, quantifying angles of rotation about a ball joint is very difficult if not next to impossible in practice.

The assembly of claim 2 further provides improvements regarding at least one of simplicity and robustness, manufacturing cost and user friendliness.

The assembly of claim 3 further provides accuracy and user friendliness in that in at least one direction a substantially pure rotation is measurable, without one or more portions also performing a translation, which requires space for manoeuvring and may introduce errors. In case all first, second and third axes intersect at or close to one point a cardan-like arrangement may be provided. Such an arrangement allows concurrent correct determination of two independent angles with respect to different, perpendicularly arranged reference directions such as reference planes. The assembly of claim 6 allows to provide the further object, preferably an elongated object such as a Kirschner wire or K-wire, with a reliably determined direction for further referencing purposes.

The assembly of claim 7 may facilitate surgery and may reduce errors in determination, since chances of forgetting and/or mixing up of values are reduced. The assembly of claims 8 and 9 improve accuracy and reliability of the surgery procedure.

### BRIEF DESCRIPTION OF FIGURES

The invention will hereafter be more fully explained with reference to the drawings showing embodiments of the invention by way of example. It should be noted that like elements are indicated with like reference numerals in the appended drawings, in which:
Fig. 1 schematically shows a human pelvis and the main anatomical planes;
Fig. 2 is a perspective view of an embodiment of an apparatus for hip joint surgery;
Figs. 3A-3C show different acetabulum bodies, here bodies for determining the opening plane of an acetabulum or a acetabular cup;
Fig. 4 shows a human pelvis and a gauge tool;
Figs. 5A-6 indicate use of the assembly for determining different angles;
Figs. 7 indicate fixing an established orientation to the patient using an elongated reference object;
Figs. 8 and 9A-9D are schematic views of alternative embodiments of the assembly;
Figs. 10-13 show different embodiments of gauge tools.

### DETAILED DESCRIPTION OF EMBODIMENTS

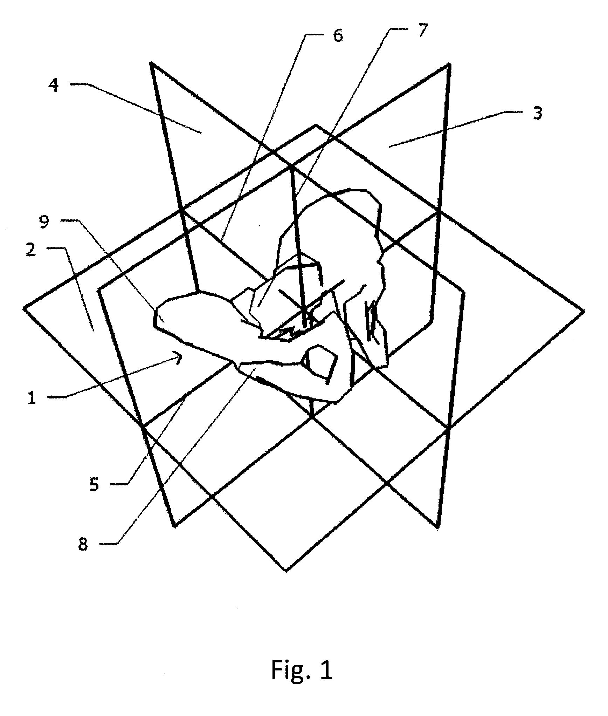

Fig. 1 shows a human pelvis 1 in reclined position with the main anatomical planes, i.e. the frontal plane or coronal plane 2, the transversal plane 3 and the sagittal plane 4, all perpendicular to each other. The intersection between the frontal plane 2 and the transversal plane 3 defines a transversal axis 5, the intersection between the frontal plane 2 and the sagittal plane 4 defines a longitudinal axis 6, the intersection between the transversal plane 3 and the sagittal plane 4 defines a sagittal axis 7. The pelvis comprises an acetabulum 8 left and right and anatomical features such as the spina iliaca anterior superior 9 left and right. In a complete hip joint the head of the femurs (not shown) reside rotatably in the acetabula.

True anteversion is rotation about the transversal axis 5, or an axis or rotation parallel to the transversal axis 5 also known as a quasi-transversal axis 5, and in the sagittal plane 4 or in a plane parallel to sagittal plane 4 known as a quasi-sagittal plane. True inclination is rotation about the sagittal axis 7, or a quasi-sagittal axis, and in the frontal plane 2 or in a quasi-frontal plane.

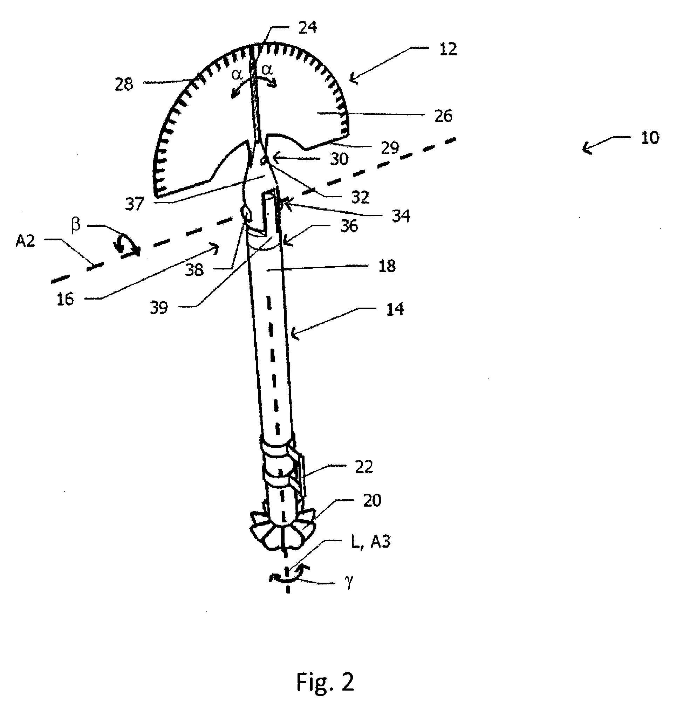

Fig. 2 shows a hip joint surgery assembly 10 comprising a goniometer 12 and a surgical tool 14. The goniometer 12 is connected to the surgical tool 14 with a joint assembly 16. The surgical tool 14 comprises a shaft portion 18, extending along an axis of extension L and an acetabulum body 20, here an acetabulum trial 20 known per se. The surgical tool 14 is provided with an optional portion 22 for holding a further object in a predetermined position with respect to the shaft portion 18.

The goniometer 12 comprises an indicator 24 and a contraindicator 26, here in the form of a blade-like body 26 provided with markings 28 and a reference structure in the form of a straight edge 29. The indicator 24 and contraindicator 26 are continuously rotatable with respect to each other over an angle α about a first axis of rotation A1 (not shown in Fig. 2) defined by a rotary joint 30 having a pivot 32. The range of angles α available in the shown embodiment, αₜₒₜ, is approximately 180 degrees (90 degrees both clockwise and anticlockwise).

The joint assembly 16 comprises a first rotary joint 34 and second rotary joint 36. The first rotary joint 34 has a first portion 37 rigidly connected to -here integral with- the indicator 24, a pivot 38 and a second portion 39. The first joint 34 is continuously rotatable over an angle β about a second axis of rotation A2 which is substantially perpendicular to the axis of extension L of the shaft portion 18. The range of angles β available in the shown embodiment, βₜₒₜ, is approximately 180 degrees (90 degrees both clockwise and counter clockwise).

The second rotary joint 36 has a first portion 39 rigidly connected to -here being identical to- the second portion 39 of the first rotary joint 34, a pivot (not visible) and a second portion rigidly connected or connectable to the shaft 18 -here being identical with the shaft 18-. The second joint 36 is continuously rotatable with an angle γ to a total angle γₜₒₜ of a full 360 degrees about a third axis of rotation A3 substantially parallel to and here coinciding with the axis of extension L of the shaft portion 18. The range of angles γ available in the shown embodiment, γₜₒₜ, is a full 360 degrees but in other embodiments it may be restricted if desired.

A suitable origin for the determination of the angles (α, β, γ) = (0, 0, 0) is defined when all three axes A1, A2, A3 are mutually perpendicular and when the indicator 24 and contraindicator 26 are in a default position, as shown in Fig. 2.

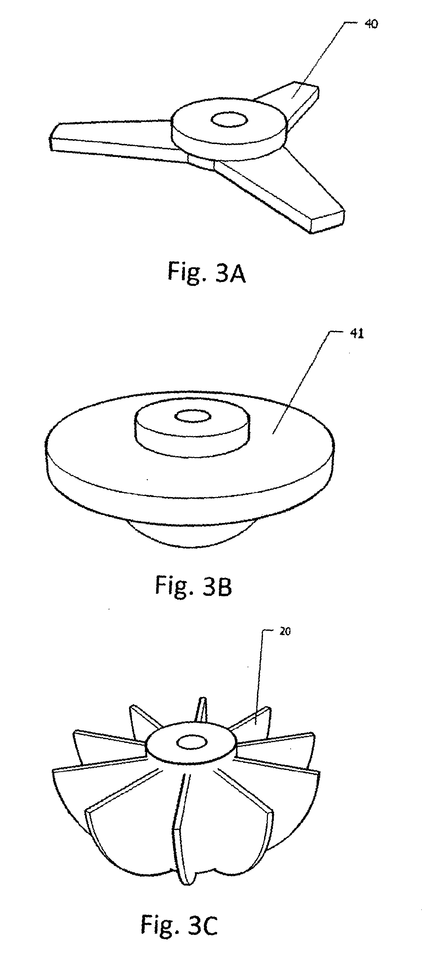

The first rotary joint 34 interconnects the goniometer 12 with the second rotary joint 36, the second rotary joint 36 interconnects the first rotary joint 34 with (the shaft portion 18 of) the surgical tool 14. The first and second axes of rotation A1, A2 and the second and third axes of rotation A2, A3 are pairwise substantially perpendicular. The rotary joints 30, 34, 36 each define a rotation in a plane substantially perpendicular to the respective axes of rotation A1, A2, A3. It should be noted that the first axis of rotation A1 may be arranged substantially parallel to the third axis of rotation A3 by rotation about the second joint for 90 degrees. Thus, (the indicator 24 of) the goniometer 12 may be manipulated in a solid angle Ω (not shown) defined by the (combination of) angles β and γ about an origin defined by the intersection of axes A2 and A3. In case the axes A2 and A3 are merely crossing without intersection, a solid angle Ω' is defined, of which the origin and extent are not clearly recognisable from the geometry of the assembly, complicating use of the device. Depending on the use of the assembly 10 and in particular the surgical tool 14 during surgery the acetabulum trial 20 may be exchanged for different objects. Examples of different acetabulum bodies are an acetabulum tripod 40 shown in Fig. 3A, for determining the position and orientation of the acetabular rim and aperture and/or holding an acetabulum cup prosthesis, an acetabulum cup measurement tool or an acetabulum cupholder 41 shown in Fig. 3B, for measuring an acetabular cup, e.g. a previously placed prosthesis, or the acetabulum trial/acetabulum measuring tool 20 shown in Fig. 2 and in more detail in Fig. 3C. Other objects may also be employed. The tool 14 and the goniometer 12 with joint assembly 16 may be detachable, such that the surgical tool 14 may be operated on with other tools, e.g. a hammer for impacting an acetabulum cup.

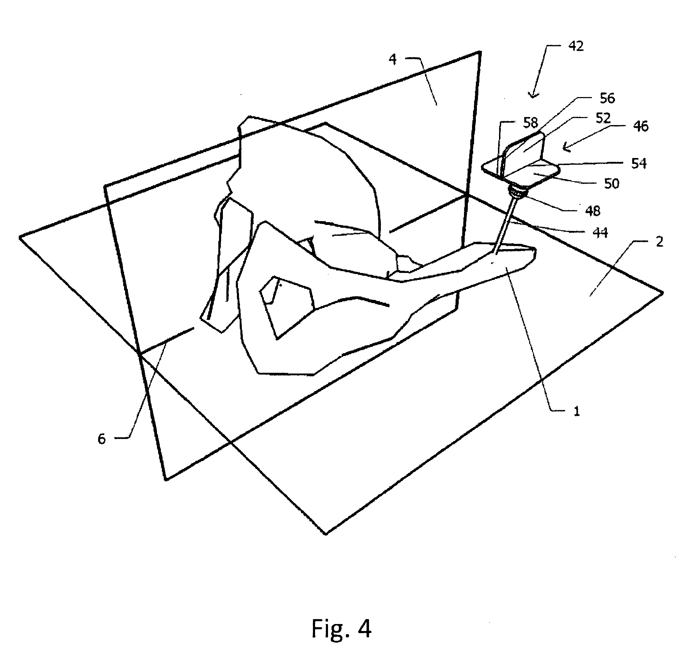

A method for performing hip surgery may comprise determination of the orientation an position of the anatomical planes of the patient, e.g. by palpating the pelvis. As explained above the correct determination of the planes during the surgical procedure is of the utmost importance. To that end, a gauge tool may be fixed to the patient, preferably fixed to the body part operated on, here the pelvis 1. An improved gauge tool 42 fixed to the left spina iliaca ant. sup. of the pelvis 1 is shown in Fig. 4.

The gauge tool 42 comprises fixing means 44, an indicator portion 46 and a joint 48. The shown fixing means 44 may be applicable through the tissue and comprises a pin 44 provided with means for fixing the pin 44 to the bone, e.g. a screw thread, one or more sharp tips etc. A plurality of fixing points prevents rotation of the pin with respect to the bone.

The joint 48 is configured to arrange and fix the indicator portion 46 in a desired position relative to the fixing means 44. A joint 48 with a one or two degrees of rotational freedom may suffice, but that requires accurate placement and/or manipulation of the fixing means 44. A joint 48 with three degrees of rotational freedom is therefore preferred, e.g. the shown ball-joint 48 with fixation means.

The indicator portion 46 comprises two plane bodies 50, 52 perpendicular to each other. To provide reference information the bodies 50 may be arranged parallel to the frontal plane 2 and the body 52 may be arranged parallel to the sagittal planes 4, to define quasi-anatomical planes. An intersection line 54 between bodies 50, 52 then is parallel to the anatomical longitudinal axis 6. The planes 50, 52 have side faces 56, 58 at straight angles such that the perimeters of the planes 50, 52 may provide further reference structures. In such orientation side faces 56, 58 at the cranial and/or caudal side of the indicator portion 46 define a quasi-transversal plane, the side face 56 defines a quasi-sagittal axis and the side face 58 defines a quasi-transversal axis. Other (anatomical) reference planes and axes may also be employed.

Using the gauge tool 42 the reference system is fixed to the relevant portion of the patient, e.g. the pelvis, thus following any (inadvertent) movement or displacement of that portion and obviating relying on non-patient-fixed reference objects such as the surgery table or the operating room.

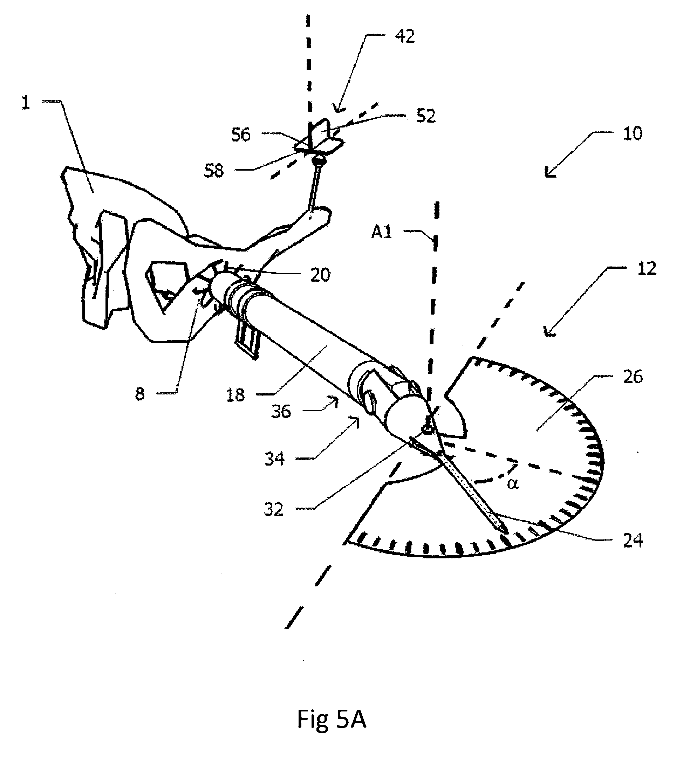

Figs. 5A-5C show determination of a desired angle of an acetabulum 8 of a pelvis 1 using the goniometer 12. The surgical tool 14 is arranged in a desired position with respect to the acetabulum 8, here by insertion of an acetabulum trial 20. The shaft 18 is brought in approximately a desired position. The goniometer 12 is manipulated by appropriately rotating the first and second joints 34, 36 such that the indicator 24 and counterindicator 26 lie in or parallel to a desired plane and the axis of rotation A1 of the goniometer 12 points in the desired direction. The angle α to be determined is found by appropriately rotating the counterindicator 26.

Fig. 5A indicates correctly determining the true inclination angle of the left hip: the goniometer 12 is oriented into a quasi-frontal plane and the axis A1 parallel to the sagittal axis 7. The correct orientation of the goniometer 12 may be checked with respect to the appropriate portions of the gauge tool 42. The first and second joints 34, 36 allow the indicator 24 to point in any direction, but the additional constraint of the direction of the axis A1 fixes the angles β, γ of the first and second joints 34, 36 to unique values. The counterindicator 26 is then rotated to bring the straight edge 29 parallel to the longitudinal axis 6 of the patient or rather the quasi-longitudinal axis 54 of the gauge tool 42. The parallel directions are indicated in Fig. 5A with dashed lines. The angle α indicated by the goniometer 12 then corresponds to the angle of inclination.

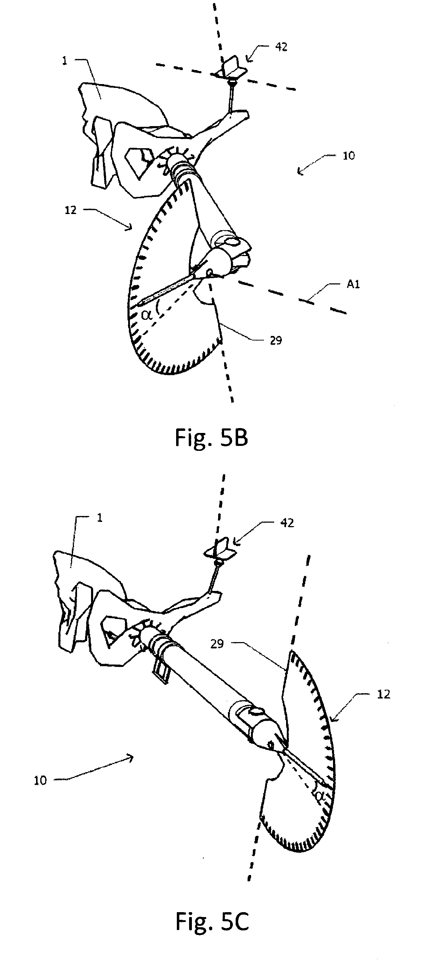

Fig. 5B indicates correctly determining the true angle of the left hip with respect to the sagittal plane: the goniometer 12 is oriented into a quasi-sagittal plane and the axis A1 parallel to the transversal axis 5. The correct orientation of the goniometer 12 may be checked with respect to the appropriate portions of the gauge tool 42. The counterindicator 26 is then rotated to bring the straight edge 29 parallel to the sagittal axis 7 of the patient or rather the quasi-sagittal axis indicated by side 56 of the gauge tool 42. The parallel directions are indicated in Fig. 5B with dashed lines. The angle α indicated by the goniometer 12 then corresponds to the true angle of the acetabulum in the sagittal plane. Using this true angle, the true inclination angle, and the anatomical planes' mutual perpendicular orientation, the true anteversion angle may be determined in straightforward manner.

The true anteversion angle can also be measured directly, by orienting the goniometer 12 in a quasi-transversal plane with the axis A1 parallel to the longitudinal axis 6 and bringing the straight edge 29 parallel to the sagittal axis 7.

Fig. 5C indicates correctly determining the planar anteversion angle of the left hip: the goniometer 12 is oriented such that the indicator 24 is aligned parallel with the axis A3 of the shaft 18, here overlapping the axis A3. Thus, the first rotary joint 34 is arranged with zero rotation and the axis A1 is arranged in a (quasi-)frontal plane, perpendicular to the main axis of the acetabulum 8 and parallel to the opening plane of the acetabulum, but the axis A1 generally will not be parallel to an anatomic axis. The counterindicator 26 is then rotated to bring the straight edge 29 parallel to the sagittal axis 7 of the patient or rather the quasi-sagittal axis indicated by side 56 of the gauge tool 42. The parallel directions are indicated in Fig. 5C with dashed lines. The angle α indicated by the goniometer 12 then corresponds to the angle of planar anteversion.

Similarly (but not shown), the planar angle of inclination may be found if the goniometer 12 is oriented such that the indicator 24 is aligned parallel with the axis A3 of the shaft 18, here overlapping the axis A3, arranging the first joint 34 with zero rotation and arranging the axis A1 in a (quasi-) transversal plane, but it generally will not be parallel to an anatomic axis. The counterindicator 26 is then rotated to bring the straight edge 29 parallel to the longitudinal axis 6 of the patient or rather the quasi-longitudinal axis 54 of the gauge tool 42. The angle α indicated by the goniometer 12 then corresponds to the angle of planar inclination.

It should be noted from the above that a goniometer with only two operative axes cannot provide the distinction between the values of true and planar anteversion and inclination. The second joint 36 allows manipulation of the goniometer 12 with respect to the shaft surgical tool 14 such that the surgical tool 14 need not be rotated with respect to the acetabulum 8 to correct the orientation of the goniometer 12 for proper determination of a desired angle. Such rotation could lead to damage of the acetabulum and/or an implanted acetabulum cup, and could lead to altering of the orientation of the shaft 18, affecting determination of the combination of the required angles (inclination, anteversion). The substantially symmetric construction of the shown embodiment assists preventing asymmetric forces on the acetabulum and/or the assembly 10 and facilitates easy use. The assembly 10 may be held with one hand and the goniometer 12 may be manipulated with another hand. The goniometer may be made light-weight for further improving user friendliness. The desired angles may be measured for information or diagnostic purposes and a predetermined orientation for arranging a prosthesis may be established and/or checked.

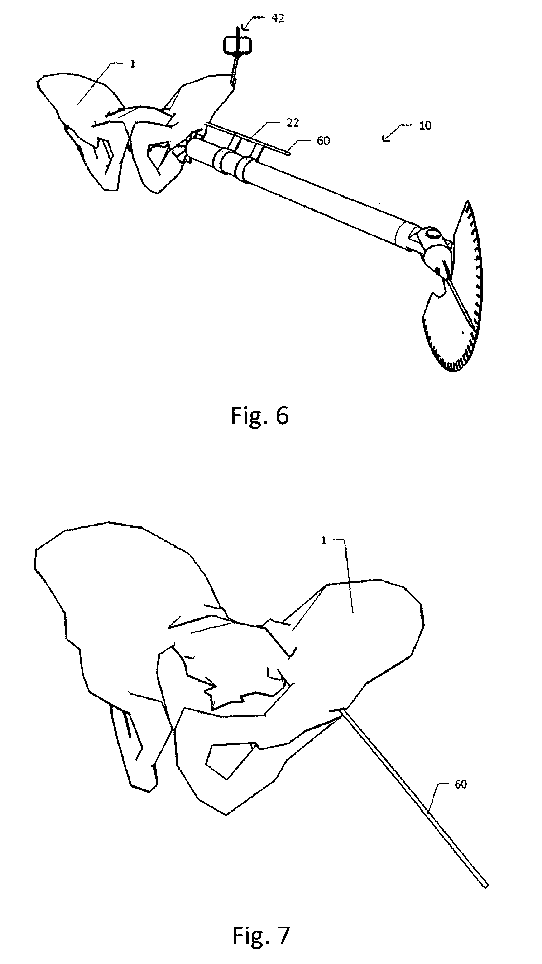

Fig. 6 shows that a once established orientation may be fixed to the patient using a reference object. With the portion 22 such reference object 60, preferably an elongated object such as a Kirschner wire 60 (or K-wire), a Steinmann pin etc. may be provided parallel to the direction of extension L of the shaft 18. The assembly 10 may then be removed, if desired the gauge tool 42 as well (Fig. 7), without significant loss of information and reliability.

Orientation of further objects during the surgery can then be compared with the reference object 60. In alternative embodiments the reference object 60 can be provided with further features. Such further features may be for (additional) marking of reference directions such as rods, rings and/or planes, and/or for subsequent guiding a direction of a surgical tool.

Succinctly put: an initial reference system of anatomic planes X, is transferred to a patient-bound (or rather, to a relevant portion of the patient to account for partial motion) reference system X' via the gauge tool 42, and a reference direction Y is determined with respect to X' via (the goniometer 12 of) the assembly 10, which in turn is transferred to a patient-bound reference object Y' (K-wire 60) for further use. Thus reducing or preventing displacement or reorientation of the patient (or the relevant portion of the patient) during a surgical procedure.

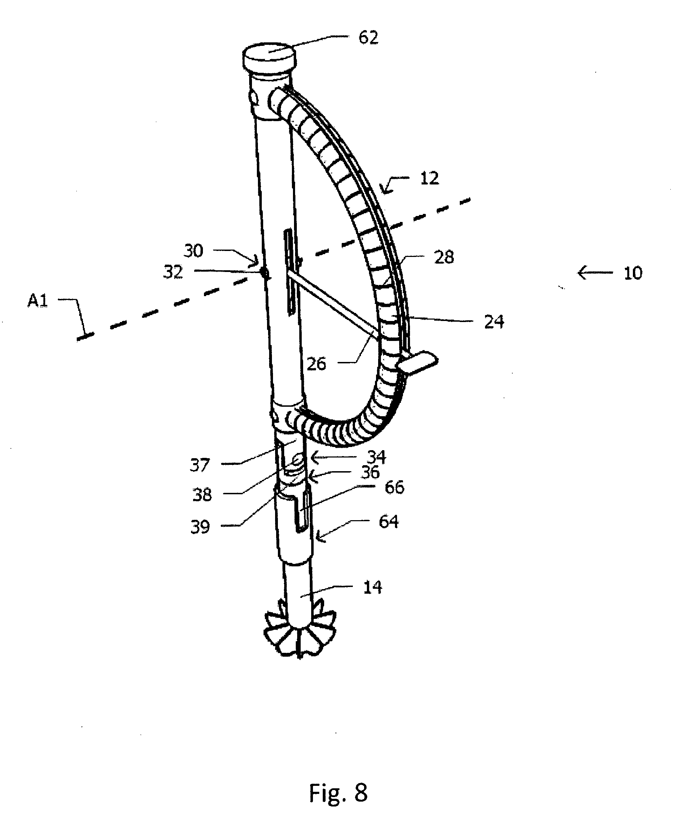

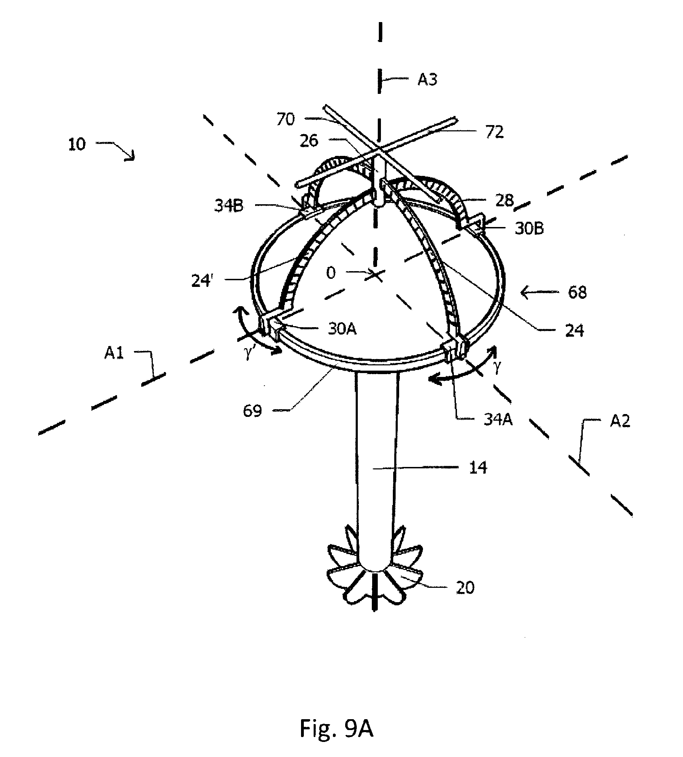

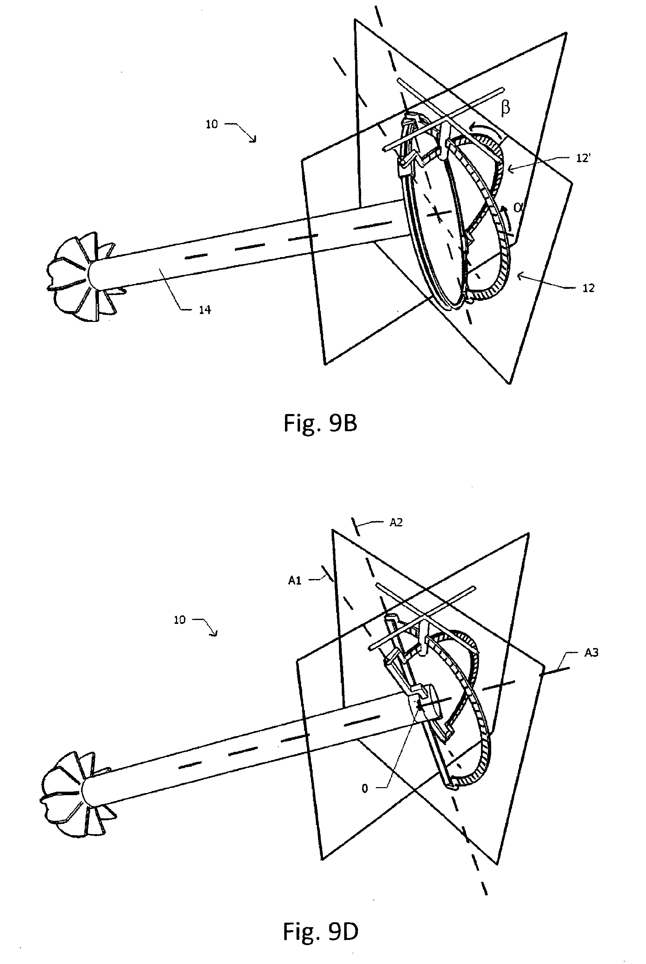

Figs. 8 and 9A-9D show second and third embodiments of an assembly 10. In the second embodiment (Fig. 8) the goniometer 12 the indicator 24 is formed as an arc in one plane perpendicular to the first axis A1 and carrying markings 28. The counterindicator 26 is formed as a pointer hand connected to the indicator. Operation of this embodiment is substantially identical to the first embodiment of Figs. 2, 5A-5C, except that the counterindicator 26 should be aligned with the relevant axis, instead of the straight edge 29. The second embodiment may further facilitate single-handed operation of the goniometer 12. The shown second embodiment comprises a top portion 62 configured as an anvil such that the assembly 10 may be used as an impactor for applying an acetabulum cup.

In a variant of the first embodiment of Figs. 2, 5A-5C, the indicator 24 may be configured to provide an anvil 62. The portion 37 may for instance be a robust rod-like object provided with a first slot accommodating the counterindicator 26 and a second slot perpendicular to the first slot for acting as an indicator 24 to read a marking 28 on the counterindicator 26.

As shown in Fig. 8 an assembly 10 with an anvil portion 62 may be provided with a sleeve portion 64 about the shaft 18 which may be fixed around the first and second joints 34, 36 for fixing these with respect to (the axis of extension L of) shaft 18. The sleeve 64 may have a recess 66 for accommodating a portion of the indicator 24 and/or counterindicator 26.

In a variant (not shown) of the second embodiment the goniometer 12 may be constructed symmetrical e.g. by providing a further arc opposite the shown indicator 24, or by forming the indicator as an arc over substantially 360 degrees.

Figs. 9A-9B show a third embodiment of an assembly 10 comprising a cardan-like configuration integrating the joint of the goniometer and the joint assembly of the first joint and the second joint, such that the axes of rotation A1, A2 and A3 intersect perpendicularly at all times at an origin O. The assembly 10 comprises a mounting portion 68 with a rail portion 69 tracing a circle segment about the origin O along the circumference of the mounting portion 68.

The joint of the goniometer 12 is formed by two joint portions 30A, 30B. The first joint 34 is likewise provided as two joint portions 34A and 34B. The second joint 36 is formed by the joint portions 30A, 30B; 34A, 34B being slidably arranged along the rail portion 69. The second joint 36 allows rotation of the mounting joint portions 30A, 30B, 34A, 34D with respect to surgical tool 14. The assembly 10 provides two goniometers 12, 12' which are usable concurrently. A first, substantially semicircular, arc-shaped indicator 24 provided with markings 28 is attached to the mounting portion 68 with rotary joints 30A, 30B providing an operative axis of rotation A1. The first indicator 24 is rotatable about an axis of rotation A2. A second, substantially semicircular, arc-shaped indicator 24'provided with markings 28 is attached to the mounting portion 68 with the rotary joints 34A, 34B providing an operative axis of rotation A2. The second indicator 24' is rotatable about an axis of rotation A1.

A counterindicator 26 is movably attached to both the first and second indicators 24, 24'. The counterindicator 26 is provided with reference structures 70, 72 which here lie in the planes of the indicators 24, 24' respectively. The counterindicator 26 is configured such that the indicators 24 and 24' are maintained in a mutual perpendicular orientation at the position of the counterindicator 26. The counterindicator 26 is movable with respect to each indicator 24, 24'; a displacement of the counterindicator 26 along the first indicator 24 corresponds to a rotation of the counterindicator 26 with respect to the origin O about the axis A1 and causes a rotation of the second indicator 24' about the associated axis A2, due to the rotary joint 34A, 34B. The position of the counterindicator 26 with respect to the second indicator 24' may remain stationary during the displacement with respect to the first indicator 24. Likewise, the counterindicator 26 may be rotated about the axis A2 and displaced with respect to the second indicator 24' and remain stationary with respect to the first indicator 24. A displacement of the counterindicator 26 to with respect to both indicators 24, 24' to determine a particular angle is enabled by the joint portions 30A, 30B; 34A, 34B, respectively, being movable with respect to the mounting portion 68. Thus the axes A1 and A2 are rotatable about the axis A3, while keeping the axes mutually pairwise perpendicular to each other; axes A1 and A2 by means of the counterindicator 26, axes A1 and A3 by the joint portions 30A, 30B and axes A2 and A3 by the joint portions 34A, 34B.

Thus, the indicator 24 is rotatable about A3 with respect to the surgical tool 14 and the operative orientation of the axis A1 of the goniometer 12 may be adjusted into any desired orientation and an angle α may be determined as described for the second embodiment.

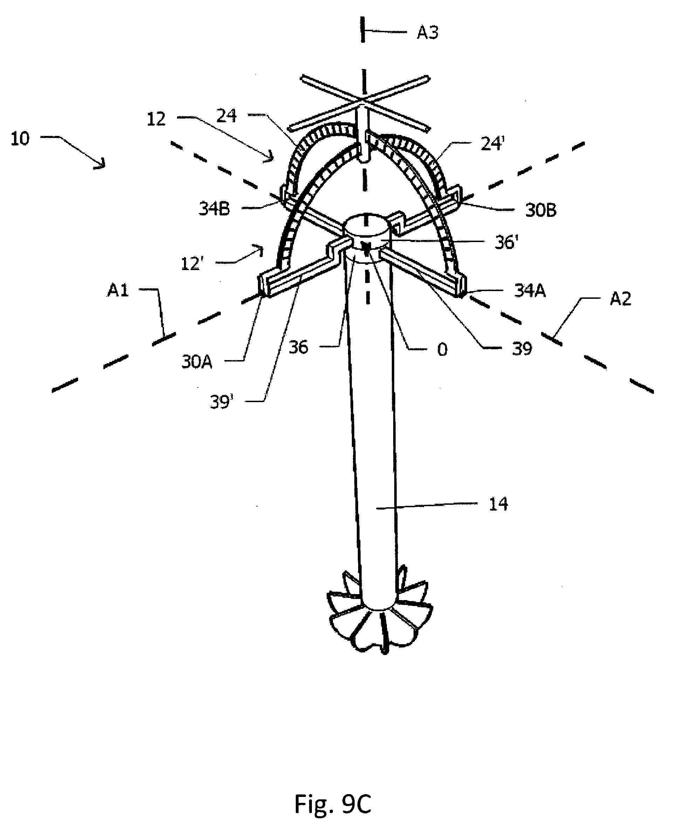

At the same time, the counterindicator 26 may be used to measure a second angle α' with respect to the second indicator 24'. Since in general a goniometer can be used with either the indicator indicating a reference direction and the counterindicator indicating the direction to be determined, but also the other way around, in which case the angle α may carry a different sign or be offset by ±90 degrees. Since further the true anteversion angle and the true inclination angle are determined with respect to mutually perpendicular planes and axes, and the operative axes A1, A2 of both goniometers 12, 12' can be arranged in perpendicular planes without introducing an offset leading to a projection, both true angles may be determined concurrently. This is indicated in Fig. 9B for relatively large angles α, β. Figs. 9C-9D show a variant of the embodiment of Figs. 9A-9B in the similar positions as Figs. 9A-9B. In Figs. 9C-9D each goniometer 12, 12' is rotatably connected to the surgical tool with an individual second joint 36, 36', respectively, attached to the joint portions 30A, 30B; 34A, 34B, respectively, with rod-like structures 39, 39'. The rod-like structures 39, 39' are configured such that the joint portions 30A, 30B; 34A, 34B are arranged in one plane, so as to provide a cardan-like arrangement with all axes A1, A2, A3 continuously intersecting at the origin O. Thus, operation of the assembly 10 is as described before. The embodiment of Figs. 9C-9D may reduce weight of the assembly 10 with respect to the embodiment of Figs. 9A-9B and the individual second joints may provide reduced friction and smoother operation than the embodiment of Figs. 9A-9B.

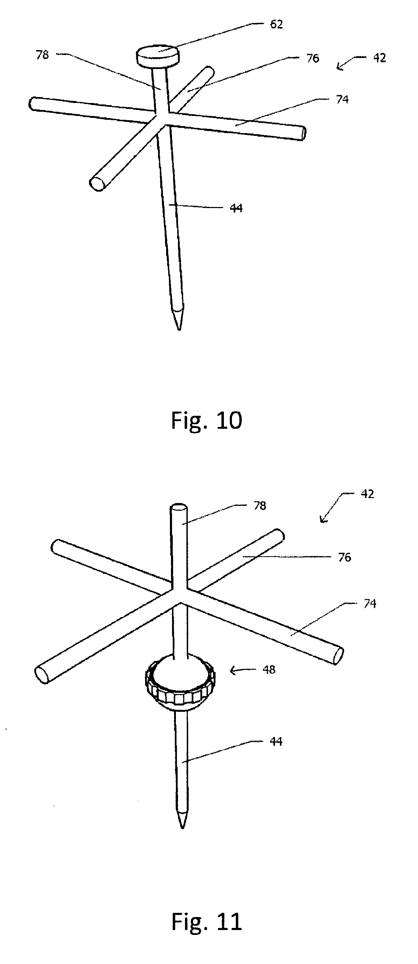

Figs. 10-13 show various embodiments of a gauge tool. Fig. 10 shows a simple embodiment of a gauge tool 42, comprising an anvil portion 62 for insertion into bone by hammering. Reference axes are indicated with rod-like structures 74, 76 and 78 attached to the fixing portion 44. With such embodiment significant care is required during fixation to ensure correct positioning.

The gauge tool 42 of Fig. 11 is a combination of the embodiments of Figs. 4 and 10. Depending on the robustness of the ball joint 48 this embodiment may be hammered into the bone or fixed via other means. This embodiment allows adjustment of the orientation of the reference structures 74-78.

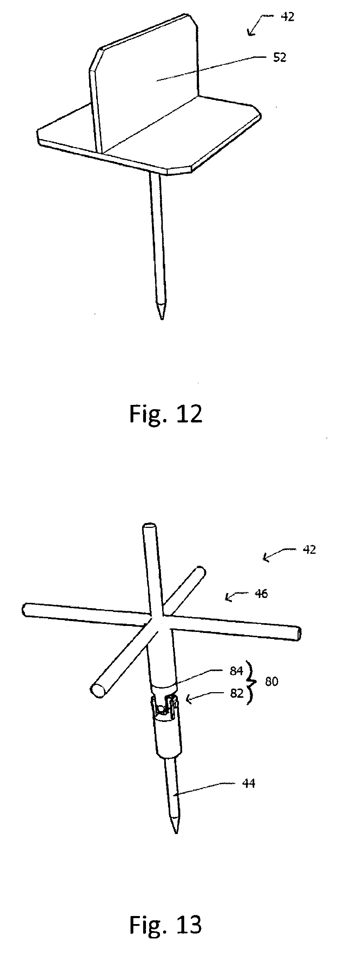

The gauge tool of Fig. 12 combines an indicator portion as in the embodiment of Fig. 4 with the simplicity of the gauge tool of Fig. 10. The plane 52 may comprise an anvil portion.

The gauge tool of Fig. 13 comprises a joint 48 in the form of a joint assembly 80 in turn comprising a cardan joint 82 and a rotary joint 84 allowing swivelling about an axis of extension of the fixing portion 44. This allows to arrange the indicator portion 46 in substantially any orientation, in which orientation the indicator portion 46 may be fixed by any suitable means, e.g. a wing nut or a quick-release skewer (not shown).

The invention is not restricted to the above described embodiments which can be varied in a number of ways within the scope of the claims. For instance, the goniometer may be formed separable from the surgical tool, e.g. as an accessory to an existing acetabulum cup impactor.

The goniometer, the surgical tool and the gauge tool may be marketed and sold separately and/or as kit of parts.

The gauge tool may be modular such that different indicator portions may be mounted to the joint of the gauge tool if so desired, damaged indicator portions and/or fixing portions be exchanged and/or facilitating sterilisation of the tool. The fixing means may comprise a screw thread, one or more sharp tips etc. A plurality of fixing points prevents rotation of the pin with respect to the bone. The pivot 32 of the goniometer 12 of the first and second embodiments may be provided with a reference structure such as a protrusion or a rod-like structure for clearer indication of the direction of its axis of rotation A1.

An assembly may comprise two separate goniometers, e.g. provided on different branch portions of a Y-shaped shaft portion 18. Such assembly may be used for educational purposes and/or facilitate concurrent measuring of plural angles.

Elements and aspects discussed for or in relation with a particular embodiment may be suitably combined with elements and aspects of other embodiments, unless explicitly stated otherwise.

## Claims

1. An assembly (10) for joint surgery, in particular hip surgery, comprising
a goniometer (12) for determining an angle for joint surgery, and
a surgical tool (14) comprising an axis of extension (L),
wherein the goniometer comprises an indicator (24) and a counterindicator (26), configured to indicate an angle (α) between at least a portion of the indicator and at least a portion of the counterindicator with respect to a first axis of rotation (A1),
**characterised in that** the goniometer is at least one of connected and connectable to the surgical tool with at least a first rotary joint (34; 34A,34B) arranged so as to be rotatable about a second axis of rotation (A2) substantially perpendicular to the axis of extension
and a second rotary joint (36, 36') arranged so as to be rotatable about a third axis of rotation (A3) substantially parallel to, preferably coinciding with, the axis of extension
such that the first axis of rotation of the goniometer is arrangable with respect to the axis of extension in three dimensions, preferably at least parallel to the axis of extension.

2. The assembly (10) of claim 1, wherein the first rotary joint (34; 34A, 34B) of the assembly comprises a first body (37) and a second body (39, 39') rotatably connected to each other and
wherein the indicator (24; 24') or the counterindicator (26; 26'), respectively, of the goniometer (12; 12') is substantially rigidly connected to the first body and the contraindicator or the indicator, respectively, of the goniometer is rotatable about the first axis of rotation (A1).

3. The assembly (10) of claim 1 or 2, wherein at least one of the first axis of rotation (A1) and the second axis of rotation (A2) of the goniometer (12) and the second axis of rotation (A2) and the third axis of rotation (A3) of the goniometer intersect.

4. The assembly (10) of any preceding claim, wherein the goniometer (12; 12') comprises means for at least temporarily fixing at least one of the first joint (34; 34A, 34B) and the second joint (36, 36') in a particular joint-angle.

5. The assembly (10) of any preceding claim, wherein the surgical tool (14) is at least one of attached or attachable to an acetabulum cup, acetabulum cup holder, an acetabulum trial, an acetabulum impactor or an acetabulum reamer.

6. The assembly (10) of any preceding claim, wherein the assembly (10) comprises means (22) for fixing a further object (60) to a body part (1) of a patient along a predetermined direction, in particular substantially parallel to the axis of extension (L) of the surgical tool (14).

7. The assembly (10) of any preceding claim, comprising a second goniometer (12') for determining a further angle for joint surgery, in particular hip surgery,
wherein the second goniometer comprises an indicator (24') and a counterindicator (26'), configured to indicate an angle (β) between at least a portion of the indicator and at least a portion of the counterindicator with respect to a first axis of rotation of the second goniometer (A2),
wherein the second goniometer is at least one of connected and connectable to the surgical tool (14) with at least a first rotary joint (30; 30A,30B) arranged so as to be rotatable about a second axis of rotation (A1) substantially perpendicular to the axis of extension (L)
and a second rotary joint (36, 36') arranged so as to be rotatable about a third axis of rotation (A3) substantially parallel to, preferably coinciding with, the axis of extension
such that the first axis of rotation of the second goniometer is arrangable with respect to the axis of extension in three dimensions.

8. The assembly (10) of any preceding claim, comprising means (42; 70,72) for concurrently indicating a plurality of predetermined reference planes.

9. The assembly (10) of any preceding claim, further comprising a gauge tool (42) in turn comprising
fixing means (44) for fixing the gauge tool to a body part (1) of a patient,
indicator means (46) for concurrently indicating a plurality of predetermined reference planes,
and means (48; 80) for fixing the indicator means in a desired position relative to the fixing means.

## Patentansprüche

1. Vorrichtung (10) zur Gelenkchirurgie, insbesondere Hüftchirurgie, mit einem Winkelmesser (12) zum Bestimmen eines Winkels bei der Gelenkchirurgie, und
einem chirurgischen Instrument (14) mit einer Verlängerungsachse (L),
wobei der Winkelmesser eine Anzeigeeinrichtung (24) und eine Gegenanzeigeeinrichtung (26) zum Anzeigen eines Winkels (α) zwischen wenigstens einem Teil der Anzeigeeinrichtung und wenigstens einem Teil der Gegenanzeigeeinrichtung in Bezug zu einer erste Rotationsachse (A1),
**dadurch gekennzeichnet, dass** der Winkelmesser mit wenigstens einem Drehgelenk (34; 34A, 34B) mit einem chirurgischen Instrument wenigstens verbunden oder verbindbar ist, so dass der Winkelmesser um eine zweite, im Wesentlichen rechtwinklig zur Verlängerungsachse verlaufende Rotationsachse (A2) rotierbar ist,
und ein zweites Drehgelenk (36, 36') derart angeordnet, dass es um eine dritte, im Wesentlichen parallel zur Verlängerungsachse, vorzugsweise zusammenfallend mit der Verlängerungsachse, verlaufende Rotationsachse (A3) rotierbar ist,
so dass die erste Rotationsachse des Winkelmessers in Bezug auf die Verlängerungsachse in drei Dimensionen, vorzugsweise zumindest parallel zur Verlängerungsachse, angeordnet werden kann.

2. Vorrichtung (10) nach Anspruch 1, wobei das erste Drehgelenk (34; 34A, 34B) der Vorrichtung einen ersten Körper (37) und einen zweiten Körper (39, 39') aufweist, welche rotierbar miteinander verbunden sind, und
wobei die Anzeigeeinrichtung (24; 24') bzw. die Gegenanzeigeeinrichtung (26; 26') des Winkelmessers (12; 12') im Wesentlichen steif mit dem ersten Körper verbunden ist und die Gegenanzeigeeinrichtung bzw. die Anzeigeeinrichtung des Winkelmessers um eine erste Rotationsachse (A1) rotiert werden kann.

3. Vorrichtung (10) nach Anspruch 1 oder 2, wobei sich wenigstens eine der ersten Rotationsachse (A1) und der zweiten Rotationsachse (A2) des Winkelmessers (12) und die zweite Rotationsachse (A2) und die dritte Rotationsachse (A3) des Winkelmessers schneiden.

4. Vorrichtung (10) nach einem der vorstehenden Ansprüche, wobei der Winkelmesser (12; 12') Mittel zum zumindest zeitweiligen Fixieren eines von dem ersten Gelenk (34; 34A, 34B) und dem zweiten Gelenk (36, 36') in einem bestimmten Gelenkwinkel aufweist.

5. Vorrichtung (10) nach einem der vorstehenden Ansprüche, wobei das chirurgische Instrument (14) mit einer Hüftgelenkspfanne, einem Hüftgelenkspfannenhalter, einer Test-Hüftgelenkspfanne, einer Hüftgelenkspfannen-Einschlagvorrichtung oder einem Hüftgelenksfräser wenigstens verbunden oder verbindbar ist.

6. Vorrichtung (10) nach einem der vorstehenden Ansprüche, wobei die Vorrichtung (10) Mittel (22) zum Befestigen eines weiteren Objekts (60) an einem Körperteil (1) eines Patienten entlang einer vorbestimmten Richtung, insbesondere im Wesentlichen parallel zur Verlängerungsachse (L) des chirurgischen Instruments (14), aufweist.

7. Vorrichtung (10) nach einem der vorstehenden Ansprüche, mit einem zweiten Winkelmesser (12') zum Bestimmen eines weiteren Winkels bei der Gelenkchirurgie, insbesondere Hüftchirurgie,
wobei der zweite Winkelmesser eine Anzeigeeinrichtung (24') und eine Gegenanzeigeeinrichtung (26') aufweist zum Anzeigen eines Winkels (β) zwischen wenigstens einem Teil der Anzeigeeinrichtung und wenigstens einem Teil der Gegenanzeigeeinrichtung in Bezug auf eine erste Rotationsachse des zweiten Winkelmessers (A2),
wobei der zweite Winkelmesser mit wenigstens einem Drehgelenk (30; 30A, 30B) mit einem chirurgischen Instrument (14) wenigstens verbunden oder verbindbar ist, so dass der zweite Winkelmesser um eine zweite, im Wesentlichen rechtwinklig zur Verlängerungsachse (L) verlaufende Rotationsachse (A1), rotierbar ist
und ein zweites Drehgelenk (36, 36') derart angeordnet ist, dass es um eine dritte, im Wesentlichen parallel zur Verlängerungsachse, vorzugsweise zusammenfallend mit der Verlängerungsachse, verlaufende Rotationsachse (A3) rotierbar ist,
so dass die erste Rotationsachse des zweiten Winkelmessers in Bezug auf die Verlängerungsachse in allen drei Raumrichtungen angeordnet werden kann.

8. Vorrichtung (10) nach einem der vorstehenden Ansprüche, mit Mitteln (42; 70, 72) zum gleichzeitigen Anzeigen einer Mehrzahl von vorbestimmten Referenzebenen.

9. Vorrichtung (10) nach einem der vorstehenden Ansprüche, mit einem Anzeigeinstrument (42), wiederum mit
Befestigungsmitteln (44) zum Befestigen des Anzeigeinstruments an einem Körperteil (1) eines Patienten,
Anzeigemitteln (46) zum gleichzeitigen Anzeigen einer Mehrzahl an vorbestimmten Referenzebenen,
und Mitteln (48; 80) zum Befestigen der Anzeigemittel in einer gewünschten Position in Bezug auf die Befestigungsmittel.

## Revendications

1. Ensemble (10) pour de la chirurgie articulaire, en particulier la chirurgie de la hanche, comprenant
un goniomètre (12) pour déterminer un angle de chirurgie articulaire, et
un outil chirurgical (14) comprenant un axe d'extension (L),
dans lequel le goniomètre comporte un indicateur (24) et un contre-indicateur (26), pouvant indiquer un angle (α) entre au moins une partie de l'indicateur et au moins une partie du contre-indicateur par rapport à un premier axe de rotation (A1),
**caractérisé en ce que**
le goniomètre est soit relié et/ou peut être relié à l'outil chirurgical avec au moins un premier joint rotatif (34 ; 34A, 34B) agencé de façon à pouvoir tourner autour d'un deuxième axe de rotation (A2) sensiblement perpendiculaire à l'axe d'extension
et un deuxième joint rotatif (36, 36') agencé de façon à pouvoir tourner autour d'un troisième axe de rotation (A3) sensiblement parallèle à l'axe d'extension, coïncidant de préférence avec celui-ci
de sorte que le premier axe de rotation du goniomètre puisse être agencé par rapport à l'axe d'extension en trois dimensions, de préférence au moins parallèle à l'axe d'extension.

2. Ensemble (10) de la revendication 1, dans lequel le premier joint rotatif (34; 34A, 34B) de l'ensemble comprend un premier corps (37) et un deuxième corps (39, 39') reliés en rotation entre eux et
où l'indicateur (24 ; 24') ou le contre-indicateur (26 ; 26') du goniomètre (12 ; 12'), respectivement, est sensiblement relié au premier corps de façon rigide et le contre-indicateur ou l'indicateur du goniomètre, respectivement, peut tourner autour du premier axe de rotation (A1).

3. Ensemble (10) de la revendication 1 ou 2, dans lequel au moins l'un du premier axe de rotation (A1) et du deuxième axe de rotation du goniomètre (12) et le deuxième axe de rotation (A2) et le troisième axe de rotation (A3) du goniomètre présentent une intersection.

4. Ensemble (10) de l'une des revendications précédentes, dans lequel le goniomètre (12 ; 12') comporte un moyen pour fixer au moins temporairement au moins l'un du premier joint (34; 34A, 34B) et du deuxième joint (36, 36') dans un angle de joint particulier.

5. Ensemble (10) de l'une des revendications précédentes, où l'outil chirurgical (14) est soit attaché ou peut être attaché à une cupule acétabulaire, un support de cupule acétabulaire, un essai acétabulaire, un impacteur acétabulaire ou un alésoir acétabulaire.

6. Ensemble (10) de l'une des revendications précédentes, dans lequel l'ensemble (10) comporte un moyen (22) pour fixer un objet supplémentaire (60) à une partie (1) du corps d'un patient le long d'une direction prédéterminée, en particulier sensiblement parallèle à l'axe d'extension (L) de l'outil chirurgical (14).

7. Ensemble (10) de l'une des revendications précédentes, comprenant un deuxième goniomètre (12') pour déterminer un angle supplémentaire pour la chirurgie articulaire, en particulier la chirurgie de la hanche,
dans lequel le deuxième goniomètre comprend un indicateur (24') et un contre-indicateur (26'), pouvant indiquer un angle (β) entre au moins une partie de l'indicateur et au moins une partie du contre-indicateur par rapport à un premier axe de rotation du deuxième goniomètre (A2),
dans lequel le deuxième goniomètre est soit relié ou peut être relié à l'outil chirurgical (14) avec au moins un premier joint rotatif (30; 30A, 30B) disposé de façon à pouvoir tourner autour d'un deuxième axe de rotation (A1) sensiblement perpendiculaire à l'axe d'extension (L)
et un deuxième joint rotatif (36, 36') agencé de façon à pouvoir tourner autour d'un troisième axe de rotation (A3) sensiblement parallèle à l'axe d'extension, coïncidant de préférence avec ce dernier
de sorte que le premier axe de rotation du deuxième goniomètre puisse être agencé par rapport à l'axe d'extension en trois dimensions.

8. Ensemble (10) de l'une des revendications précédentes, comprenant un moyen (42 ; 70, 72) pour indiquer de manière simultanée une pluralité de plans de référence prédéterminés.

9. Ensemble (10) de l'une des revendications précédentes, comprenant en outre un outil jauge (42) comprenant à son tour
un moyen de fixation (44) pour fixer l'outil jauge à une partie (1) du corps d'un patient,
un moyen indicateur (46) pour indiquer une pluralité de plans de référence prédéterminés de manière simultanée,
et un moyen (48, 80) pour fixer le moyen indicateur dans une position désirée par rapport au moyen de fixation.
